# EUROPEAN PATENT SPECIFICATION

(11) **EP 1 297 742 B1**
(45) Date of publication and mention of the grant of the patent: **06.04.2005**
(21) Application number: 02078771.9
(22) Date of filing: 16.09.2002
(51) Int. Cl.: A01J 5/007, A01J 5/013, A01J 5/01, A01J 5/017

(54) **A method of collecting measurement data during automatically milking an animal**
Verfahren zur Messwerterfassung während des automatischen Melkens eines Tieres
Méthode de collecte de données de mesure pendant la traite automatique d'un animal

(30) Priority: 28.09.2001 NL 1019061
(43) Date of publication of application: 02.04.2003
(73) Proprietor: Lely Enterprises AG, 6300 ZUG (CH)
(72) Inventor: Vijverberg, Helena Geralda Maria, 3141 GM Maassluis (NL); Espada Aventin, Elena, 2627 AD Delft (NL)
(74) Representative: Corten, Maurice Jean F.M.

(56) References cited:
- EP-A- 0 639 327
- EP-A- 0 764 403
- EP-A- 1 000 535
- WO-A-97/47187
- WO-A-99/31965
- US-A- 5 743 209

## Description

The present invention relates to a method of collecting measurement data during automatically milking of a dairy animal according to the preamble of claim 1, see e.g. document EP-A-0 764 403.

Such a method in which milking takes place by means of a milking robot is known. In such a method i.a. the value of the conductivity of the milk produced by the dairy animal can be measured. In this situation the value of the conductivity is thus constituted by the value of a variable in relation to the dairy animal as mentioned in the preamble of claim 1. In dependence on the value of the conductivity it may be decided whether or not the milk obtained is suitable for being processed further. However, it has appeared that the known method sometimes draws a wrong conclusion on the basis of the measured conductivity, so that e.g. suitable milk is not used for being processed further, but is discharged.

It is i.a. an object of the invention to provide a method of milking a dairy animal by means of which the decision whether or not milk obtained is suitable for being processed further can be taken in an accurate manner.

According to the invention, for that purpose a method of the above-described type comprises the measures according to the characterizing part of claim 1. The invention is based on the insight that the measured value of the variable depends on the measured period, also called interval, even when the condition of the dairy animal remains unchanged. By repeatedly varying, according to the invention, the admission criterion in such a manner that periods of time with different values are obtained, it is possible to obtain automatically measurement signals belonging to the various periods. By period is meant in particular a period of time measured by a clock or a number of dairy animals having been milked between the two successive milking runs. Alternatively also other variables may be considered as periods, such as e.g. the quantity of milk produced between the two successive milking runs. By not only using one single measured value but a measurement pattern, it is possible to take a more accurate decision whether or not the milk obtained should be processed further. The average measurement pattern may excellently be used for determining deviations from this average pattern, which may be an indication that the condition of the dairy animal is different from normal or that the milk produced by the dairy animal is different from normal. Such an average measurement pattern appears to provide per animal a more accurate indication of the deviation than a predetermined reference value. Especially when the average is a so-called progressive average, i.e. an average over e.g. the last ten milking runs (another number is possible as well), it is possible to take an accurate decision whether or not the milk obtained should be processed further.

In an embodiment of a method according to the invention the average measurement pattern is stored per period, so that it is possible to store the measurement patterns (respectively measured values) per period and to compare them with momentary measurement patterns respectively measured values.

In a further embodiment of a method according to the invention a momentary measurement pattern of a milk variable in a measured period is compared with the stored measurement pattern of the milk variable for the same period, and there is issued a comparison signal indicative of the comparison result. It is thus possible, when the device is provided with a milk line system comprising a number of lines and with at least one device controlled by the comparison signal for guiding milk flowing through the milk line system to a relevant line, to discharge automatically unsuitable milk or to convey suitable milk for being processed further.

There is preferably issued, in dependence on the comparison signal, a warning to the manager of the device, e.g. in the form of a sound signal.

The step of measuring a value of a variable in relation to the dairy animal preferably comprises measuring the intensity of at least one wavelength band, in particular in the visible wavelength range, of the milk obtained from the dairy animal, the variable being the intensity of the wavelength band. In particular the intensity of the separate colours in the milk obtained from the separate udder quarters is established. In this embodiment the variable is thus constituted by the colour of the milk obtained.

The step of measuring a value of a variable in relation to the dairy animal preferably comprises measuring the flow of the milk obtained during the milking run. Preferably the flow of the milk obtained from the separate udder quarters is measured.

The step of measuring a value of a variable in relation to the dairy animal preferably comprises measuring the conductivity of the milk obtained during the milking run. Preferably the conductivity of the milk obtained from the separate udder quarters is measured.

In a still further embodiment of a method according to the invention the step of measuring a value of a variable in relation to the dairy animal comprises measuring the temperature of the milk obtained during the milking run. Preferably the temperature of the milk obtained from the separate udder quarters is measured.

In another further embodiment of a method according to the invention the step of measuring a value of a variable in relation to the dairy animal comprises measuring the quantity of a component of the milk obtained during the milking run, such as fat, protein, urea, bacteria, sugars, free fatty acids, germs, etc. Preferably the quantity of a component of the milk obtained from the separate udder quarters is measured.

In another further embodiment of a method according to the invention the step of measuring a value of a variable in relation to the dairy animal comprises measuring the quantity of the milk obtained during the milking run. Preferably the quantity of the milk obtained from the separate udder quarters is measured.

In a still further embodiment of a method according to the invention the step of measuring a value of a variable in relation to the dairy animal comprises measuring the activity of the dairy animal during the milking run.

The invention will be explained hereinafter in further detail with reference to an embodiment shown in the drawing, in which:
Figure 1 is a schematic view of a device for milking a cow, provided with a colour sensor measuring system, and
Figure 2 is a schematic view of a milking box with a milking robot provided with means for measuring a variable in relation to the cow.

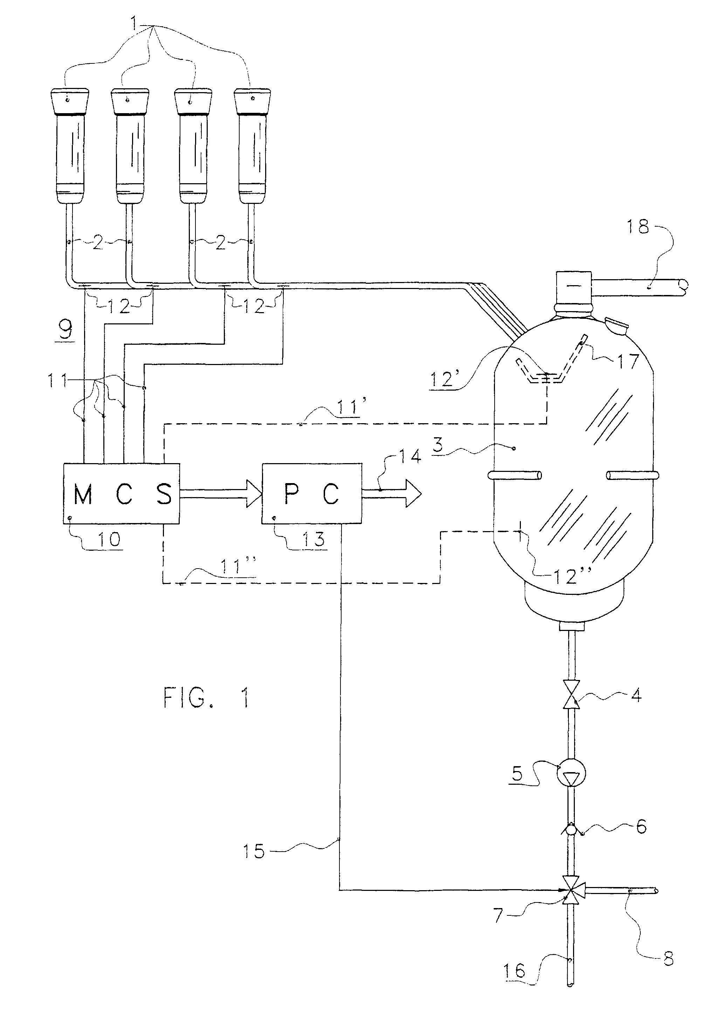

Figure 1 shows four teat cups 1 to be connected to the teats of an animal to be milked, the milk discharge lines 2 of said teat cups 1 debouching into a milk glass 3. To the milk glass 3 there is further connected a vacuum line 18 for the purpose of applying a vacuum in the milk glass 3 itself, in the milk discharge lines 2 and in the teat cups 1, said vacuum being required for keeping the teat cups connected to the teats of the animal, for enabling milking and for separating milk and air present therein from each other in the milk glass 3. From the milk glass 3 the milk obtained is discharged via a valve 4, a pump 5, a non-return valve 6 and a three-way valve 7 through a line 8 to a not further shown milk tank.

Figure 1 further shows a colour sensor measuring system 9, said measuring system comprising a colour intensity processing unit (MCS) 10 to which four sensors 12 are connected via glass fibre cables 11. Said sensors 12 are disposed in the milk lines 2 for establishing the intensity of a number of defined colours in the milk and for supplying signals representing these intensities to the processing unit 10. As a colour sensor measuring system may be used the Modular Color Sensor system CS1 of Stracon Messsysteme GmbH, Im Camisch 10, Kahla. The sensors used in this system are sensitive to frequencies in frequency bands for red (R), green (G) and blue (B). Therefore there are issued three signals per measurement, which may be considered as intensity values for these three colours.

Although until now the opinion prevailed that for milk of a constant composition these three intensity values have a fixed mutual relation, said relation depending i.a. on the impurities and components in the milk, it has appeared that with certain dairy animals the relation between the three intensity values depends on the interval, in other words depends on the period between two successive milking runs.

The colour intensity processing unit (MCS) 10 comprises a computer (PC) 13 (shown in the figure separately from the colour intensity processing unit (MCS) for the sake of clearness), in which for each animal to be milked there is a file in which all data required for milking a relevant animal are stored.

At each milking run also the obtained three intensity values of the relevant colours in the milk are stored. These intensity values stored at each milking run thus form the so-called historical intensity values. The progressive average may be determined from the historical intensity vales obtained for a certain animal during a defined number of the last milking runs carried out. Upon averaging milking runs with equal intervals should be used. The intensity values obtained at a next milking run with an equal interval may be compared with this progressive average, i.e. the last obtained intensity value of each of the three colours may be compared with the corresponding intensity value belonging to that interval, recorded in the computer as a progressive average. In other words, the intensity values are compared both mutually and with corresponding intensity values recorded during one or more previous milking runs with an equal interval. This comparison process takes place in the computer 13 which also functions as a comparing device. Subsequently the results of this comparison process may be displayed on a displaying device in such a manner that the presence of certain substances, such as impurities, in the milk can be read directly therefrom. These results may be supplied via the line 14 to a screen or to a printer.

Instead of determining the progressive average of the intensity values for each of the colours, it is also possible to determine in another manner for each colour a calibration value, such as in particular a reference pattern, respectively a lower threshold pattern or an upper threshold pattern. It is possible to apply calibration values which could hold for the milk obtained from all the animals or from a group of animals. In that case it will not be necessary to dispose a sensor 12 in each of the milk discharge lines 2, but an overflow reservoir 17 may be disposed in the milk glass 3, in which overflow reservoir there is provided such a sensor 12' which is connected to the processing unit 10 via a glass fibre cable shown by a "dashed" line 11'. As a further alternative a sensor 12" may be disposed in the lower part of the milk glass 3. Also in the latter case said sensor has to be connected to the processing unit 10 via a glass fibre cable 11".

However, in all situations it applies that, when inadmissible quantities of undesired substances appear to be present in the milk, the computer 13 issues a signal over the line 15 to the three-way valve 7, via which three-way valve 7 and the discharge line 16 connected thereto the milk containing these undesired substances may be discharged separately.

When for example blood has come into the milk, the intensity value issued by the sensor 12 for the colour red, will be higher than when no blood is present in the milk. This intensity value will then be higher than the progressive average established on the basis of the historical intensity values or higher than the calibration value applied (of course in dependence on the comparison with values belonging to the same interval). Also when there are no impurities in the milk, alterations in the concentration of substances normally being present in the milk may still be established. When for example the fat content of the milk changes in the course of the lactation period, then the mutual relation of the three intensity values established during each milking run changes as well.

Because the composition of the milk is different for different animals, which is even visually perceptible from the colour, the intensity values for the three colours will have a mutually different ratio for different animals. Therefore it is advantageous to determine the intensity values for each animal separately at each milking run and to compare them with calibration values or, in particular, with progressive averages established for this specific animal (and belonging to the same interval).

An example of the dependence of the measured colour intensity on the interval, said dependence having been proved clearly by means of the above-mentioned colour sensor measuring system, is given hereinafter. It has further appeared that this dependence is reproducible. For a particular cow it has appeared that the intensity of the blue frequency band rises in a particular manner when the period of time, the interval, (or the number of cows having been milked) increases. It has further appeared that the intensity of the green frequency band shows a certain, slight fall at an increasing interval. The intensity of the red frequency band showed a certain slight rise. For this cow the total sum of the intensities appeared to rise to a maximum value at an increasing interval and to fall via a particular pattern at a further increasing interval. The value of the intensity in the red frequency band reduced by the value of the blue frequency band appeared to show with this cow a falling pattern at an increasing interval, whereas the quotient of the intensity in the red frequency band and the intensity in the green frequency band rose to a maximum value at an increasing interval and remained constant at a further increase of the interval. It will be obvious that upon comparing the milk obtained from this cow, at each interval there has to be taken a different reference value or pattern to decide whether or not the milk obtained is suitable for being processed further.

It has further appeared that the colour intensity may differ per quarter, so that it is advantageous to compare the data per animal, per quarter, per interval, in order to be able to decide whether or not milk obtained from a quarter should be processed further.

It has further appeared that the flow of the milk obtained during the milking run depends on the interval. Also here, to be able to take a correct decision whether or not the milk obtained should be processed further, the measured flow values have to be compared with the reference value for that interval. It is noticed that a flow sensor for measuring the flow of the milk obtained during the milking run is known per se. In particular the flow sensor measures the flow of the milk obtained from the separate udder quarters. For the above-mentioned cow it has appeared that the flow rises at an increasing interval.

It has further appeared that the conductivity of the milk obtained for the mentioned cow rises at an increasing interval. A conductivity meter for measuring the conductivity of the milk obtained during the milking run, in particular per quarter, may then be used to take a correct decision whether or not the milk obtained (possibly per quarter) should be processed further.

It has further appeared that the temperature of the milk obtained for the mentioned cow rises at an increasing interval. In that situation a thermometer may be used for measuring the temperature of the milk obtained during the milking run, in particular for measuring the temperature of the milk obtained from the separate udder quarters, in order to take a correct decision whether or not the milk obtained (possibly per quarter) should be processed further.

Moreover it has appeared that for the mentioned cow the fat content of the milk obtained falls according to a certain curve at an increasing interval. Also for other components there appears to be a dependence between the quantity and the interval. A component meter for measuring the quantity of a component of the milk obtained during the milking run, such as fat, protein, urea, bacteria, sugars, free fatty acids, germs, etc., in particular the components of the milk obtained from the separate udder quarters, may then be used for taking a correct decision whether or not the milk obtained (possibly per quarter) should be processed further.

Besides it has appeared that for the mentioned cow the milk yield increases at an increasing interval. A quantity meter for measuring the quantity of the milk obtained during the milking run, in particular for measuring the quantity of the milk obtained from the separate udder quarters, may then be used in order to take a correct decision whether or not the milk obtained (possibly per quarter) should be processed further.

Research has also revealed that the activity of the mentioned cow, e.g. determined by means of a step counter, depends on the interval. Moreover it appears that an increased activity may indicate an udder inflammation, as a result of which the cow experiences pain and tries to kick off the teat cups during connection of the teat cups. Udder inflammation affects, as known, the quality of the milk, so that the activity may be used to determine the quality of the milk. The activity may also be determined in another manner, e.g. by measuring the heartbeat of the cow.

The above-mentioned relations have not only been found with a particular cow, but all cows appear to produce milk of which the measurable variables depend on the interval. Moreover cows also appear to show an interval-dependent activity. It will be obvious that the exact nature of that dependence can be determined by measurement.

As already mentioned above for colour intensity measurement, especially a measured measurement pattern (also called measured curve) of the variable appears to be adapted to decide during the milking run whether or not milk obtained should be processed further. This applies in particular to the pattern of colour, conductivity and flow during a milking run, although the other above-mentioned variables also show a pattern during the milking run, which pattern may be used for obtaining a correct decision whether or not milk obtained is suitable for being processed further.

In this situation an averaging device may determine the average of a measurement pattern of a milk variable and use this average as a reference pattern. Besides, other reference patterns are possible as well (e.g. an upper threshold pattern and/or a lower threshold pattern).

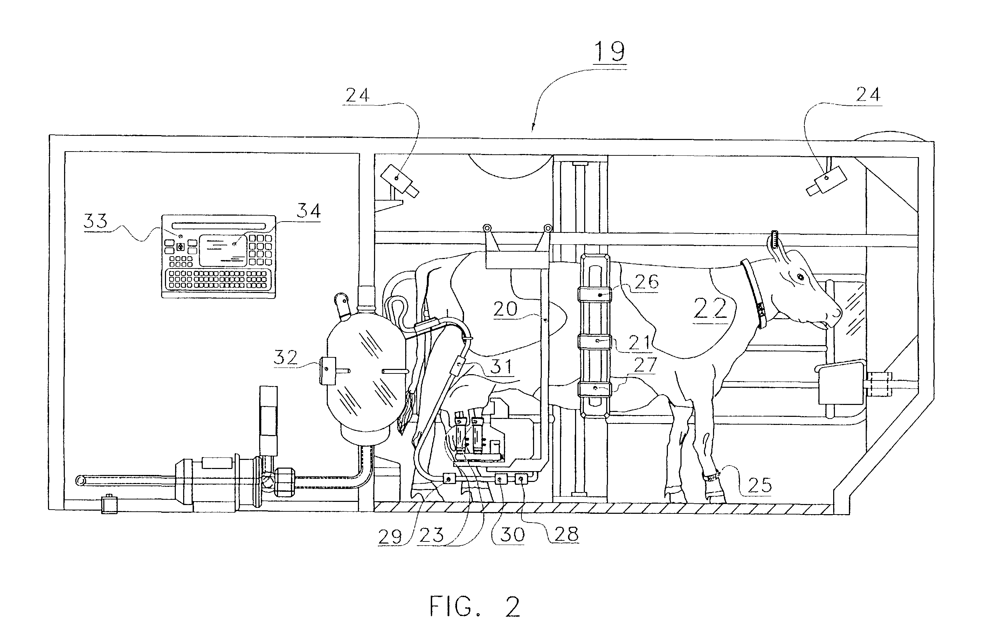

Figure 2 shows schematically a milking box 19 with a milking robot 20, to which a cow gets access or in which a cow is actually milked in dependence on an admission criterion, which is known per se. According to the invention this admission criterion is varied in such a manner, preferably periodically, that milking runs for a cow at different intervals are obtained, so that relevant measurement data and reference values can be used for determining whether or not milk obtained is suitable for being processed further. In the milking box various measuring devices are present for measuring variables in relation to the cow.

For example the heart beat may be measured by means of a band 21 including a heart beat meter around the leg or the abdomen of the cow 22. Alternatively or additionally a heart beat meter known per se may be provided on the cow 22 near a place where an artery is located, in this connection the udder or an ear of the cow may be taken into consideration. A suitable heart monitoring system is for example obtainable at Polar Electro Oy, Helsinki, Finland. Alternatively a heart beat meter may be included in at least one of the teat cups 23.

In the milking box 19 there may be disposed one or more cameras 24 for observing and measuring the activity of the cow 22. The video pictures are analysed by movement recognition equipment known per se for determining activity parameters such as stepping, kicking and the like. To that end the picture is compared per cow 22 with stored historical data regarding the cow 22. Also in this situation it applies, as mentioned above, that the historical data used for the comparison relate to the same interval.

There may further be provided a step counter 25, a muscle contraction meter 26 and/or a muscle vibration meter 27 for determining the activity of the cow 22.

A flow sensor 28 measures the flow of the milk obtained during a milking run. A conductivity meter 29 measures the conductivity of the milk obtained during a milking run. A thermometer 30 measures the temperature of the milk obtained during a milking run. A component meter 31 measures the components, e.g. protein and fat, in the milk obtained during the milking run, and the milk yield is measured by a quantity meter 32 or yield meter.

All these measurement data are transmitted to or read by a processing device 33 comprising a computer having a memory. Besides the measurement data the processing device 33 also stores the period of time elapsed since the same animal has been milked. Alternatively the processing device stores the number of cows having been milked. To that end the processing device 33 comprises a clock (not explicitly shown, but implicitly present in the computer) for determining the period of time between two successive milking runs of the dairy animal. Alternatively the processing device comprises a counter for counting the number of cows having been milked. In the memory of the computer of the processing device 33 reference values or reference patterns are stored per interval, per animal or per group of animals, possibly per quarter, and per milk variable, respectively these reference values or reference patterns are generated by the system itself. The processing device 33 comprises a (non-shown) comparing device for comparing the measured value of the variable with the stored reference values. The comparing device issues a comparison signal, the value of which depends on the comparison result, and is thus indicative of the comparison result. This comparison signal may be displayed on a displaying device, such as a screen 34. As described above, the comparison signal may also be used for controlling a valve or the like, so that the milk obtained will be processed further or not. Should the comparison signal indicate a deviation, then it is also possible for the comparison signal to control a device for generating a warning (such as e.g. a loudspeaker) for issuing a signal (e.g. a sound) which is perceptible by a manager of the device.

It will be obvious that the measured values may be used separately, but that also combinations of measured values of different variables may be used for determining whether or not milk should be processed further (or for determining whether the condition of a dairy animal is within the standards). Thus a weight factor may be given to certain parameters for combining the measured values and/or comparison results obtained in a desired manner. It will further be obvious that in view of the number of different admission criterions known to a person skilled in the art, an explicit description thereof has been omitted for the sake of simplicity. It will be clear for a person skilled in the art that the relevant criterion should be altered repeatedly for obtaining measurement data (measurement signals) at different intervals, e.g. 1, 1.5, 2, 2.5, ..., hours.

As described, Figure 2 shows a side view of a milking box 19 with a cow 22 present therein. The milking box 19 is provided with a milking robot 20 with teat cups 23 which are automatically connected to the teats of the cow 22 by means of the milking robot 20. Near the front side of the milking box 19 there is further disposed a feeding trough to which concentrate may be supplied in metered quantities. Other elements of the milking box and the robot are not shown in the figure for the sake of clearness.

## Claims

1. A method of collecting measurement data during automatically milking a dairy animal by means of a device provided with a milking box (19) with a milking robot (20), said method comprising the steps of:
determining the period between two successive milking runs of the dairy animal,
measuring a value of a variable in relation to the dairy animal,
issuing a measurement signal indicative of the measured value,
admitting a dairy animal to the milking box (19) in dependence on an admission criterion,
repeatedly varying the admission criterion in such a manner that periods with different values are obtained,
obtaining automatically measurement signals belonging to the various periods, and
storing the measurement signals per period in a memory,
**characterized in that** the method comprises the step of measuring during the entire course of the milking run the value of the milk variable for obtaining a measurement pattern of the milk variable, the step of storing the measurement pattern in a memory, and the step of determining the average of a measurement pattern of a milk variable belonging to the same period.

2. A method as claimed in claim 1, **characterized in that** the method comprises the step of storing the average measurement pattern per period.

3. A method as claimed in claim 1 or 2, **characterized in that** the method comprises the step of storing a reference pattern per period.

4. A method as claimed in claim 2 or 3, **characterized in that** the method comprises the step of comparing a momentary measurement pattern of a milk variable in a measured period with the stored measurement pattern of the milk variable for the same period, and of issuing a comparison signal indicative of the comparison result.

5. A method as claimed in claim 4, **characterized in that** the method comprises the step of controlling the milk flow in dependence on the comparison signal.

6. A method as claimed in claim 4 or 5, **characterized in that** the method comprises the step of generating a warning in dependence on the comparison signal.

7. A method as claimed in any one of the preceding claims, **characterized in that** the step of determining the period between two successive milking runs of the dairy animal comprises measuring the period of time between two successive milking runs by means of a clock.

8. A method as claimed in any one of claim 1 to 6, **characterized in that** the step of determining the period between two successive milking runs of the dairy animal comprises counting the number of dairy animals having been milked between the two successive milking runs.

9. A method as claimed in any one of the preceding claims, **characterized in that** the step of measuring a value of a variable in relation to the dairy animal comprises measuring the intensity of at least one wavelength band, in particular in the visible wavelength range, of the milk obtained from the dairy animal, the variable being the intensity of the wavelength band.

10. A method as claimed in claim 9, **characterized in that** the intensity of the separate colours in the milk obtained from the separate udder quarters is established.

11. A method as claimed in any one of the preceding claims, **characterized in that** the step of measuring a value of a variable in relation to the dairy animal comprises measuring the flow of the milk obtained during the milking run.

12. A method as claimed in claim 11, **characterized in that** the flow of the milk obtained from the separate udder quarters is measured.

13. A method as claimed in any one of the preceding claims, **characterized in that** the step of measuring a value of a variable in relation to the dairy animal comprises measuring the conductivity of the milk obtained during the milking run.

14. A method as claimed in claim 13, **characterized in that** the conductivity of the milk obtained from the separate udder quarters is measured.

15. A method as claimed in any one of the preceding claims, **characterized in that** the step of measuring a value of a variable in relation to the dairy animal comprises measuring the temperature of the milk obtained during the milking run.

16. A method as claimed in claim 15, **characterized in that** the temperature of the milk obtained from the separate udder quarters is measured.

17. A method as claimed in any one of the preceding claims, **characterized in that** the step of measuring a value of a variable in relation to the dairy animal comprises measuring the quantity of a component of the milk, such as fat, protein, urea, bacteria, sugars, free fatty acids, germs, etc. obtained during the milking run.

18. A method as claimed in claim 17, **characterized in that** the quantity of the components of the milk obtained from the separate udder quarters is measured.

19. A method as claimed in any one of the preceding claims, **characterized in that** the step of measuring a value of a variable in relation to the dairy animal comprises measuring the quantity of the milk obtained during the milking run.

20. A method as claimed in claim 19, **characterized in that** the quantity of the milk obtained from the separate udder quarters is measured.

21. A method as claimed in any one of the preceding claims, **characterized in that** the step of measuring a value of a variable in relation to the dairy animal comprises measuring the activity of the dairy animal during the milking run.

## Patentansprüche

1. Verfahren zum Sammeln von Meßdaten während des automatischen Melkens eines milchgebenden Tieres mittels einer Vorrichtung, die eine Melkbox (19) mit einem Melkroboter (20) aufweist, wobei das Verfahren folgende Verfahrensschritte umfaßt:
Ermitteln des Zeitraumes zwischen zwei aufeinanderfolgenden Melkdurchgängen des milchgebenden Tieres,
Messen eines Wertes einer das milchgebende Tier betreffenden Variablen,
Ausgeben eines den gemessenen Wert anzeigenden Meßsignals,
Zulassen eines milchgebenden Tieres zu der Melkbox (19) in Abhängigkeit von einem Zulassungskriterium,
wiederholtes Verändern des Zulassungskriteriums derart, daß Zeiträume mit unterschiedlichen Werten erzielt werden,
automatisches Erzeugen von zu den verschiedenen Zeiträumen gehörenden Meßsignalen,
Speichern der Meßsignale für jeden Zeitraum in einem Speicher,
**dadurch gekennzeichnet, daß** das Verfahren den Verfahrensschritt des Messens des Wertes der Melkvariablen während der gesamten Dauer des Melkdurchganges zur Erzielung eines Meßmusters der Melkvariablen, den Verfahrensschritt des Speicherns des Meßmusters in einem Speicher und den Verfahrensschritt des Ermittelns des Durchschnitts eines Meßmusters einer Melkvariablen desselben Zeitraumes umfaßt.

2. Verfahren nach Anspruch 1,
**dadurch gekennzeichnet, daß** das Verfahren den Verfahrensschritt des Speicherns des Durchschnittsmeßmusters für jeden Zeitraum umfaßt.

3. Verfahren nach Anspruch 1 oder 2,
**dadurch gekennzeichnet, daß** das Verfahren den Verfahrensschritt des Speicherns eines Bezugsmusters für jeden Zeitraum umfaßt.

4. Verfahren nach Anspruch 2 oder 3,
**dadurch gekennzeichnet, daß** das Verfahren den Verfahrensschritt des Vergleichens eines momentanen Meßmusters einer Melkvariablen in einem gemessenen Zeitraum mit dem gespeicherten Meßmuster der Melkvariablen für denselben Zeitraum und des Ausgebens eines Vergleichssignals zur Anzeige des Vergleichsergebnisses umfaßt.

5. Verfahren nach Anspruch 4,
**dadurch gekennzeichnet, daß** das Verfahren den Verfahrensschritt des Steuerns des Milchflusses in Abhängigkeit von dem Vergleichssignal umfaßt.

6. Verfahren nach Anspruch 4 oder 5,
**dadurch gekennzeichnet, daß** das Verfahren den Verfahrensschritt des Erzeugens einer Warnung in Abhängigkeit von dem Vergleichssignal umfaßt.

7. Verfahren nach einem der vorhergehenden Ansprüche,
**dadurch gekennzeichnet, daß** der Verfahrensschritt des Ermittelns des Zeitraumes zwischen zwei aufeinanderfolgenden Melkdurchgängen des milchgebenden Tieres das Messen des Zeitraumes zwischen zwei aufeinanderfolgenden Melkdurchgängen mittels einer Uhr umfaßt.

8. Verfahren nach einem der Ansprüche 1 bis 6,
**dadurch gekennzeichnet, daß** der Verfahrensschritt des Ermittelns des Zeitraumes zwischen zwei aufeinanderfolgenden Melkdurchgängen des milchgebenden Tieres das Zählen der Anzahl von milchgebenden Tieren umfaßt, die zwischen den beiden aufeinanderfolgenden Melkdurchgängen gemolken wurden.

9. Verfahren nach einem der vorhergehenden Ansprüche,
**dadurch gekennzeichnet, daß** der Verfahrensschritt des Messens eines Wertes einer das milchgebende Tier betreffenden Variablen das Messen der Intensität mindestens eines Wellenlängenbandes, insbesondere im sichtbaren Wellenlängenbereich, der von dem milchgebenden Tier gewonnenen Milch umfaßt, wobei die Variable die Intensität des Wellenlängenbandes ist.

10. Verfahren nach Anspruch 9,
**dadurch gekennzeichnet, daß** die Intensität der einzelnen Farben in der von den einzelnen Eutervierteln gewonnenen Milch ermittelt wird.

11. Verfahren nach einem der vorhergehenden Ansprüche,
**dadurch gekennzeichnet, daß** der Verfahrensschritt des Messens eines Wertes einer das milchgebende Tier betreffenden Variablen das Messen des während des Melkdurchganges erzielten Milchflusses umfaßt.

12. Verfahren nach Anspruch 11,
**dadurch gekennzeichnet, daß** der von den einzelnen Eutervierteln gewonnene Milchfluß gemessen wird.

13. Verfahren nach einem der vorhergehenden Ansprüche,
**dadurch gekennzeichnet, daß** der Verfahrensschritt des Messens eines Wertes einer das milchgebende Tier betreffenden Variablen das Messen der Leitfähigkeit der während des Melkdurchganges gewonnenen Milch umfaßt.

14. Verfahren nach Anspruch 13,
**dadurch gekennzeichnet, daß** die Leitfähigkeit der von den einzelnen Eutervierteln gewonnenen Milch gemessen wird.

15. Verfahren nach einem der vorhergehenden Ansprüche,
**dadurch gekennzeichnet, daß** der Verfahrensschritt des Messens eines Wertes einer das milchgebende Tier betreffenden Variablen das Messen der Temperatur der während des Melkdurchganges gewonnenen Milch umfaßt.

16. Verfahren nach Anspruch 15,
**dadurch gekennzeichnet, daß** die Temperatur der von den einzelnen Eutervierteln gewonnenen Milch gemessen wird.

17. Verfahren nach einem der vorhergehenden Ansprüche,
**dadurch gekennzeichnet, daß** der Verfahrensschritt des Messens eines Wertes einer das milchgebende Tier betreffenden Variablen das Messen der Menge eines Bestandteiles, wie z. B. Fett, Protein, Harnstoff, Bakterien, Zucker, freie Fettsäuren, Keime usw., der während des Melkdurchganges gewonnenen Milch umfaßt.

18. Verfahren nach Anspruch 17,
**dadurch gekennzeichnet, daß** die Menge der Bestandteile der von den einzelnen Eutervierteln gewonnenen Milch gemessen wird.

19. Verfahren nach einem der vorhergehenden Ansprüche,
**dadurch gekennzeichnet, daß** der Verfahrensschritt des Messens eines Wertes einer das milchgebende Tier betreffenden Variablen das Messen der Menge der während des Melkdurchganges gewonnenen Milch umfaßt.

20. Verfahren nach Anspruch 19,
**dadurch gekennzeichnet, daß** die Menge der von den einzelnen Eutervierteln gewonnenen Milch gemessen wird.

21. Verfahren nach einem der vorhergehenden Ansprüche,
**dadurch gekennzeichnet, daß** der Verfahrensschritt des Messens eines Wertes einer das milchgebende Tier betreffenden Variablen das Messen der Aktivität des milchgebenden Tieres während des Melkdurchganges umfaßt.

## Revendications

1. Procédé de collecte de données de mesure pendant la traite automatique d'un animal laitier au moyen d'un dispositif pourvu d'un box de traite (19) avec un robot de traite (20), ledit procédé comprenant les étapes consistant à :
déterminer la période entre deux opérations de traite successives de l'animal laitier,
mesurer une valeur d'une variable relative à l'animal laitier,
émettre un signal de mesure indicatif de la valeur mesurée,
admettre un animal laitier dans le box de traite (19) en fonction d'un critère d'admission,
modifier le critère d'admission de façon répétée de telle manière que des périodes avec différentes valeurs soient obtenues ;
obtenir des signaux de mesure automatiques appartenant aux différentes périodes ; et
stocker les signaux de mesure par période dans une mémoire, **caractérisé en ce que** le procédé comprend l'étape consistant à mesurer pendant l'ensemble de la durée de l'opération de traite la valeur de la variable de lait pour obtenir un modèle de mesure de la variable de lait, l'étape consistant à stocker le modèle de mesure dans une mémoire, et l'étape consistant à déterminer la moyenne d'un modèle de mesure d'une variable de lait appartenant à la même période.

2. Procédé selon la revendication 1, **caractérisé en ce que** le procédé comprend l'étape consistant à stocker le modèle de mesure moyen par période.

3. Procédé selon la revendication 1 ou 2, **caractérisé en ce que** le procédé comprend l'étape consistant à stocker un modèle de référence par période.

4. Procédé selon la revendication 2 ou 3, **caractérisé en ce que** le procédé comprend l'étape consistant à comparer le modèle de mesure momentané d'une variable de lait dans une période mesurée au modèle de mesure stocké de la variable de lait pour la même période, et à émettre un signal de comparaison indicatif du résultat de comparaison.

5. Procédé selon la revendication 4, **caractérisé en ce que** le procédé comprend l'étape consistant à contrôler le débit de lait en fonction du signal de comparaison.

6. Procédé selon la revendication 4 ou 5, **caractérisé en ce que** le procédé comprend l'étape consistant à générer une alarme en fonction du signal de comparaison.

7. Procédé selon l'une quelconque des revendications précédentes, **caractérisé en ce que** l'étape consistant à déterminer la période entre deux opérations de traite successives de l'animal laitier comprend la mesure de la période de temps entre deux opérations de traite successives au moyen d'une horloge.

8. Procédé selon l'une quelconque des revendications 1 à 6, **caractérisé en ce que** l'étape consistant à déterminer la période entre deux opérations de traite successives de l'animal laitier comprend le comptage du nombre d'animaux laitiers ayant été traits entre les deux opérations de traite successives.

9. Procédé selon l'une quelconque des revendications précédentes, **caractérisé en ce que** l'étape consistant à mesurer une valeur d'une variable relative à l'animal laitier comprend la mesure de l'intensité d'au moins une bande de longueur d'onde, en particulier dans la gamme de longueurs d'onde visible, du lait obtenu de l'animal laitier, la variable étant l'intensité de la bande de longueur d'onde.

10. Procédé selon la revendication 9, **caractérisé en ce que** l'intensité des couleurs distinctes dans le lait obtenu des quartiers de pis distincts est établie.

11. Procédé selon l'une quelconque des revendications précédentes, **caractérisé en ce que** l'étape consistant à mesure une valeur d'une variable relative à l'animal laitier comprend la mesure du débit du lait obtenu pendant l'opération de traite.

12. Procédé selon la revendication 11, **caractérisé en ce que** le débit du lait obtenu des quartiers de pis distincts est mesuré.

13. Procédé selon l'une quelconque des revendications précédentes, **caractérisé en ce que** l'étape consistant à mesurer une valeur d'une variable relative à l'animal laitier comprend la mesure de la conductivité du lait obtenu pendant l'opération de traite.

14. Procédé selon la revendication 13, **caractérisé en ce que** la conductivité du lait obtenu des quartiers de pis distincts est mesurée.

15. Procédé selon l'une quelconque des revendications précédentes, **caractérisé en ce que** l'étape consistant à mesurer une valeur d'une variable relative à l'animal laitier comprend la mesure de la température du lait obtenu pendant l'opération de traite.

16. Procédé selon la revendication 15, **caractérisé en ce que** la température du lait obtenu des quartiers de pis distincts est mesurée.

17. Procédé selon l'une quelconque des revendications précédentes, **caractérisé en ce que** l'étape consistant à mesurer une valeur d'une variable relative à l'animal laitier comprend la mesure de la quantité d'un composant du lait, tel que les matières grasses, les protéines, l'urée, les bactéries, les sucres, les acides gras libres, les germes, etc., obtenu pendant l'opération de traite.

18. Procédé selon la revendication 17, **caractérisé en ce que** la quantité des composants du lait obtenu des quartiers de pis distincts est mesurée.

19. Procédé selon l'une quelconque des revendications précédentes, **caractérisé en ce que** l'étape consistant à mesurer une valeur d'une variable relative à l'animal laitier comprend la mesure de la quantité du lait obtenue pendant l'opération de traite.

20. Procédé selon la revendication 19, **caractérisé en ce que** la quantité du lait obtenue des quartiers de pis distincts est mesurée.

21. Procédé selon l'une quelconque des revendications précédentes, **caractérisé en ce que** l'étape consistant à mesurer une valeur d'une variable relative à l'animal laitier comprend la mesure de l'activité de l'animal laitier pendant l'opération de traite.
